# Europäisches Patentamt

# European Patent Office

# Office européen des brevets

(19)

(11) Veröffentlichungsnummer: **0 175 226**
**B1**

(12) # EUROPÄISCHE PATENTSCHRIFT

(45) Veröffentlichungstag der Patentschrift:
04.11.87

(51) Int. Cl.⁴: **C 07 C 143/38**

(21) Anmeldenummer: **85111252.4**

(22) Anmeldetag: **06.09.85**

(54) **Verfahren zur Herstellung von Alkali- und Erdalkalisalzen der Benzaldehyd-2,4-disulfonsäure.**

(30) Priorität: **17.09.84 DE 3434079**

(43) Veröffentlichungstag der Anmeldung:
**26.03.86 Patentblatt 86/13**

(45) Bekanntmachung des Hinweises auf die Patenterteilung:
**04.11.87 Patentblatt 87/45**

(84) Benannte Vertragsstaaten:
**CH DE FR GB IT LI**

(56) Entgegenhaltungen:
**DE-C-98 321**

(73) Patentinhaber: **HOECHST AKTIENGESELLSCHAFT,
Postfach 80 03 20, D-6230 Frankfurt am Main 80
(DE)**

(72) Erfinder: **Metz, Hans Joachim, Dr., Reiterweg 8,
D-6148 Heppenheim (DE)**

EP 0 175 226 B1

LIBER, STOCKHOLM 1987

## Beschreibung

Die Erfindung bezieht sich auf ein Verfahren zur Herstellung von Alkali- und Erdalkalisalzen der Benzaldehyd-2,4-disulfonsäure aus 2,4-Dichlorbenzaldehyd durch Umsetzung mit einem Alkali- oder Erdalkalisulfit und/oder -hydrogensulfit in Gegenwart von Wasser.

Benzaldehyd-2,4-disulfonsäure und ihre Salze stellen wertvolle technische Produkte dar und finden beispielsweise Verwendung in der galvanischen Industrie oder bei der Produktion von Triphenylmethanfarbstoffen.

Zur Herstellung von Benzaldehyd-2,4-disulfonsäure und ihren Salzen sind im wesentlichen zwei Wege bekannt:

a: Seitenkettenoxidation von Toluol-2,4-disulfonsäure mit Mangansalzen,

b: Umsetzung von 2,4-Dichlorbenzaldehyd mit einem Sulfit und/oder Hydrogensulfit.

Das Verfahren nach a ist mit dem Nachteil schwermetallhaltiger, säurebelasteter Abwässer behaftet und deshalb nach Möglichkeit zu vermeiden.

Der Stand der Technik des Verfahrens nach b ist durch die deutschen Reichspatente DRP 98 321, DRP 91 818 und DRP 88 952 gegeben.

So wird im DRP 88 952 die Umsetzung von 2-Chlorbenzaldehyd mit einer wäßrigen Natriumhydrogensulfit-Lösung in einem geschlossenen Gefäß bei Reaktionstemperaturen von 190-200°C und Reaktionszeiten von 8 h zum Natriumsalz der Benzaldehyd-2-sulfonsäure beschrieben. Analog erhält man gemäß dem DRP 91 818 aus 2,5-Dichlorbenzaldehyd das Natriumsalz der 5-Chlorbenzaldehyd-2-sulfonsäure. Schließlich wird im DRP 98 321 ein analoges Verfahren zur Gewinnung einer wäßrigen Lösung des Dinatriumsalzes der Benzaldehyd-2,4-disulfonsäure aus 2,4-Dichlorbenzaldehyd bei Reaktionstemperaturen von 190-200°C und Reaktionszeiten von 9 - 10 h beschrieben. Die Substanz wurde dabei nicht isoliert, und es fehlen Angaben zur Ausbeute.

Bei Durchführung der Reaktion unter den im Beispiel des DRP 98 321 genannten Bedingungen wird ein großer Anteil an Nebenprodukten gebildet. Man erhält dunkel gefärbte heterogene Reaktionsmischungen, aus denen nur mit erheblichem Aufwand stark verunreinigtes Produkt mit einer Ausbeute von 30-60 % d.Th. isoliert werden kann. Daher bestand die Aufgabe, ein Verfahren zur Herstellung von Alkalisalzen der Benzaldehyd-2,4-disulfonsäure zu entwickeln, das in reproduzierbarer Weise das gewünschte Produkt in wirtschaftlichen Ausbeuten liefert.

Es wurde nun gefunden, daß überraschenderweise entgegen der Lehre der oben zitierten Patente reproduzierbare Ergebnisse mit höherer Ausbeute erzielt werden, wenn die Reaktionstemperatur unter 180°C liegt. Weiterhin war überraschend, daß trotz der niedrigen Temperatur eine deutlich kürzere Reaktionszeit als nach dem Verfahren des DRP 98 321 vorteilhaft ist.

Gegenstand der Erfindung ist demnach ein Verfahren zur Herstellung eines Alkali- oder Erdalkalisalzes der Benzaldehyd-2,4-disulfonsäure aus 2,4-Dichlorbenzaldehyd durch Umsetzung mit einem Alkali- oder Erdalkalisulfit und/oder -hydrogensulfit in Gegenwart von Wasser, dadurch gekennzeichnet, daß die Reaktionstemperatur bei 140-180°C, vorzugsweise bei 160-170°C, und die Reaktionszeit unter 7 Std., vorzugsweise bei 2 bis 4 Std., liegt.

Der 2,4-Dichlorbenzaldehyd wird in zweckmäßiger Weise mit einer wäßrigen Lösung des Sulfits und/oder Hydrogensulfits gleichen Kations in einem geschlossenen Gefäß unter Rühren erhitzt.

Geeignet sind Sulfite und Hydrogensulfite der Alkali- und Erdalkalimetalle, besonders die des Natriums und Kaliums. Die geeignete Konzentration der wäßrigen Lösung hängt teilweise von der Löslichkeit der jeweiligen Sulfite und Hydrogensulfite ab. Beispielsweise sollte im Fall des Natriumsulfits das Massenverhältnis Sulfit zu Wasser bevorzugt den Wert 0,33 nicht überschreiten (z. B. 0,11 - 0,33) und besonders bevorzugt bei 0,22 - 0,25 liegen.

Die Sulfite und/oder Hydrogensulfite werden zweckmäßig in einer Menge von 2 bis 2,5 mol, vorzugsweise 2,05 bis 2,15 mol, pro mol 2,4-Dichlorbenzaldehyd eingesetzt.

Die Isolierung des Produkts erfolgt in einfacher Weise nach dessen Kristallisation und Entfernung von überschüssigem Sulfit. Zwecks Ausbeutesteigerung ist es vorteilhaft, zunächst einen Teil des Reaktionswassers abzudestillieren - vorzugsweise wird auf 65 % der Einwaage eingeengt - und anschließend die Kristallisation durch Abkühlung einzuleiten. Überschüssiges Sulfit kann nach üblichen Methoden entfernt werden, beispielsweise durch Verkochen mit Schwefelsäure oder durch Oxidation zu Sulfat. Bevorzugte Methode ist die Oxidation mit wäßriger Hypochlorit-Lösung nach dem Kristallisationsschritt. Die Abtrennung der gebildeten Kristalle des Produkts gelingt in einfacher Weise durch Zentrifugieren, kann aber auch durch Filtrieren erfolgen.

Vorteile des neuen Verfahrens gegenüber dem zitierten Stand der Technik sind in der Reproduzierbarkeit, hohen Ausbeute, Energie-Ersparnis infolge stark verkürzter Reaktionszeit und der wesentlich gesteigerten Raum-Zeit-Ausbeute begründet.

Die nachstehenden Beispiele dienen der Erläuterung der Erfindung. Die Prozentangaben beziehen sich auf das Gewicht.

## Beispiel 1

175 g (1 mol) 2,4-Dichlorbenzaldehyd werden zusammen mit einer Lösung von 260 g (2,06 mol) Natriumsulfit in 1100 g Wasser 2,5 h auf 170°C erhitzt. Danach wird auf 65 % der Einwaage eingeengt und bei 5°C mit ca. 200 g Natriumhypochloritlösung (13 % NaOC1) oxidiert. Durch Zentrifugieren erhält man 361 g Produkt mit einem Gehalt von 73,9 % an Dinatriumsalz der Benzaldehyd-2,4-disulfonsäure, entsprechend 86 % d. Th..

**Beispiel 2**

175 g (1 mol) 2,4-Dichlorbenzaldehyd werden zusammen mit einer Lösung von 260 g (2,06 mol) Natriumsulfit in 1190 g Wasser 5 h auf 170°C erhitzt. Es wird auf 65 % der Einwaage eingeengt und bei 5°C mit ca. 200 g Natriumhypochlorit-Lösung (13 % NaOCl) oxidiert. Durch Zentrifugieren erhält man 310 g Produkt mit einem Gehalt von 75,4 % an Dinatriumsalz der Benzaldehyd-2,4-disulfonsäure, entsprechend 75,4 % d.Th..

**Vergleichsbeispiel**

Man verfährt bis auf folgende Unterschiede wie in Beispiel 1 beschrieben. Die Reaktionstemperatur wird 10 Std. auf 195°C gehalten. Vor dem Kristallisationsschritt ist eine Behandlung der Reaktionsmischung mit Aktivkohle erforderlich. Man erhält 170 g Produkt mit einem Gehalt von ca. 63,5 % an Dinatriumsalz der Benzaldehyd-2,4-disulfonsäure, d.h. 34,8 % d.Th., bezogen auf 2,4-Dichlorbenzaldehyd.

**Patentansprüche**

1. Verfahren zur Herstellung eines Alkali- oder Erdalkalisalzes der Benzaldehyd-2,4-disulfonsäure aus 2,4-Dichlorbenzaldehyd durch Umsetzung mit einem Alkali- oder Erdalkalisulfit und/oder -hydrogensulfit in Gegenwart von Wasser, dadurch gekennzeichnet, daß die Reaktionstemperatur bei 140-180°C und die Reaktionszeit unter 7 Std. liegt.

2. Verfahren nach Anspruch 1, dadurch gekennzeichnet, daß die Reaktionstemperatur bei 160-170°C liegt.

3. Verfahren nach einem der Ansprüche 1 oder 2, dadurch gekennzeichnet, daß die Reaktionszeit bei 2 - 4 Std. liegt.

4. Verfahren nach einem der Ansprüche 1 bis 3, dadurch gekennzeichnet, daß das Sulfit und/oder Hydrogensulfit in einer Menge von 2 - 2,5 mol pro mol 2,4-Dichlorbenzaldehyd eingesetzt wird.

5. Verfahren nach einem der Ansprüche 1 bis 4, dadurch gekennzeichnet, daß eine wäßrige Lösung von Natriumsulfit mit einem Massenverhältnis Natriumsulfit/Wasser von 0,11 - 0,33 verwendet wird.

6. Verfahren nach Anspruch 2, dadurch gekennzeichnet, daß die Reaktionszeit 2 - 4 Std.

beträgt und 2,05 - 2,15 mol Sulfit pro mol Ausgangsstoff eingesetzt wird.

**Claims**

1. A process for the preparation of an alkali metal or alkaline earth metal salt of benzaldehyde-2,4-disulfonic acid from 2,4-dichlorobenzaldehyde by reaction with an alkali metal or alkaline earth metal sulfite and/or hydrogensulfite in the presence of water, characterized in that the reaction temperature is 140-180°C and the reaction time is less than 7 hours.

2. The process as claimed in claim 1, characterized in that the reaction temperature is 160-170°C.

3. The process as claimed in one of claims 1 or 2, characterized in that the reaction time is 2-4 hours.

4. The process as claimed in one of claims 1 to 3, characterized in that the sulfite and/or hydrogensulfite is used in a quantity of 2-2.5 mol per mol 2,4-dichloro-benzaldehyde.

5. The process as claimed in one of claims 1 to 4, characterized in that an aqueous solution of sodium sulfite is used with a mass ratio of sodium sulfite/water of 0.11-0.33.

6. The process as claimed in claim 2, characterized in that the reaction time is 2-4 hours and 2.05-2.15 mol of sulfite is used per mol of starting material.

**Revendications**

Procédé de préparation d'un sel de métal alcalin ou de métal alcalino-terreux de l'acide formyl-1 benzène-disulfonique-2,4 à partir du dichloro-2,4 benzaldéhyde, par réaction avec un sulfite et/ou un hydrogénosulfite de métal alcalin ou de métal alcalino-terreux, en présence d'eau, procédé caractérisé en ce que la température réactionnelle est comprise entre 140 et 180°C et en ce que la durée de la réaction est inférieure à 7 heures.

2. Procédé selon la revendication 1 caractérisé en ce que la température réactionnelle est comprise entre 160 et 170°C.

3. Procédé selon l'une des revendications et 2, caractérisé en ce que la durée de la réaction est comprise entre 2 et 4 heures.

4. Procédé selon l'une quelconque des revendications 1 à 3, caractérisé en ce que le sulfite et/ou l'hydrogénosulfite sont mis en jeu en une quantité de 2 à 2,5 mol par mole de dichloro-2,4 benzaldéhyde.

5. Procédé selon l'une quelconque des revendications 1 à 4, caractérisé en ce qu on utilise une solution aqueuse de sulfite de sodium avec un rapport massique du sulfite de sodium à l'eau compris entre 0,11 et 0,33.

6. Procédé selon la revendication 2 caractérisé en ce que la durée de la réaction est de 2 à 4 heures et en ce qu on met en jeu de 2,05 à 2,15 mol de sulfite par mole du corps de départ.